# EUROPEAN PATENT APPLICATION

(11) **EP 1 813 600 A1**
(43) Date of publication of application: **01.08.2007**
(21) Application number: 05825290.9
(22) Date of filing: 16.11.2005
(51) Int. Cl.: C07C 229/26, C11D 1/46, A61K 31/221

(54) **N-N-ACYLOXYPROPYL LYSINE METHYL ESTER- AND N,N-BIS(N- ACYLOXYPROPYL)LYSINE METHYL ESTER-TYPE COMPOUNDS AND USE THEREOF AS SURFACE-ACTIVE AGENTS WITH AN ANTIMICROBIAL ACTIVITY**

(30) Priority: 19.11.2004 ES 200402797
(71) Applicant: Consejo Superior de Investigaciones Cientificas, 28006 Madrid (ES)
(72) Inventor: PÉREZ MUÑOZ, Lourdes, 08034 Barcelona (ES); PINAZO GASSOL, Aurora, 08034 Barcelona (ES); INFANTE MARTINEZ-PARDO, María Rosa, 08034 Barcelona (ES); ANGELET SUBIRATS, Marta, 08034 Barcelona (ES)
(74) Representative: Ungria Lopez, Javier
(86) International application number: PCT/ES2005/070158
(87) International publication number: WO 2006/056636

(57) **Abstract**

The invention relates to novel compounds having an amphiphilic character (cationic, anionic, amphoteric and non-ionic), derivatives of n acyloxypropyl-type lysine amino acid according to general formula (I), which are intended to be used in the food, pharmaceutical and cosmetic industries as surface-active agents having a self-aggregating capacity and antimicrobial properties. Variations in activity are a function of the ionic nature of the final molecule, the number of fatty chains and the length thereof. The aforementioned products are prepared using a chemical synthesis method. The intermediate and final products are purified by means of liquid/liquid and liquid/solid extractions, crystallisations, cationic exchange chromatography and normal phase chromatography.

## Description

### SECTOR OF THE ART

Surface-active agents are organic molecules of an amphiphilic nature having broad and versatile use in the food, pharmaceutical and cosmetic industries. Their chemical structure is part polar or hydrophilic, of an ionic (anionic, cationic and amphoteric) or non-ionic nature, and part apolar or hydrophobic, normally one or two hydrocarbon chains.

The compounds forming the object of this patent are structurally analogous to cationic compounds of the type 1-0-1- arginyl ester or 3-o-monoacylglycerides or 1-o-1- arginyl ester 2,3 -o-diacylglycerides (WO 0158860), which present emulsifying or dispersant properties and, due to being cationic, antimicrobial activity. Given said structural similarity, the present derivatives of lysine will be able to be applied as antimicrobial cationic surface-active agents with emulsifying, dispersant and solubilising properties and/or controlled release agents.

### STATE OF THE ART

Current environmental demands regarding chemicals make it essential to investigate into novel noncontaminating products. In recent years, there has been growing interest in the preparation and study of surface-active agents based on natural products such as sugars, sterols, amino acids and fatty acids (Laughlin, R.G., Fu, Y.-C., Wireko, F.C. Scheibel, J.J., Munyon, R.L., Novel Surfactants, Holmerg, K. (ed.), Marcel-Dekker, New York, 1998; M. Brink, J. Novel Surfactants, Holmerg, K. (ed.), Macrcel-Dekker, New York, 1998). One of the strategies currently existing for achieving ecologically acceptable surface-active agents is to prepare molecules whose molecular structure mimics that of natural surface-active agents: lipo-amino acids, phospholipids and glycerolipids (J.H. Fendler 1989 "Membrane Mimetic Chemistry" John Wiley & Son). These surface-active agents are environmentally acceptable since they are in general innocuous and easily biodegradable (Infante, M.R., Pérez, P., Pinazo, A., Clapés. P., Morán, M.C., Angelet, M., Garcia, M.T., Vinardell, M.P. C.R. Chimie, 2004, 7, 583).

The surface-active agents derived from amino acids are very interesting compounds on account of their multifunctionality and innocuousness (Takehara, M. Colloids and Surfaces, 1989, 38, 149; Selve, C., Mansuy, L., Allouch, M., J. Chem. Res. 1992, 22, 401; Tsushima, R. Proceedings 4th World Surfactants Congress, 1996, 1, 43; Infante, M.R., Pérez, P., Pinazo, A., Clapés. P., Morán, M.C., Angelet, M., Garcia, M.T., Vinardell, M.P. C.R. Chimie, 2004, 7, 583). These characteristics have been responsible for the fact that, in the last few years, the synthesis and study of properties have been carried out on a wide variety of surface-active agents derived from amino acids, of an ionic, cationic, non-ionic and amphoteric nature (Takehara, M. Colloids and Surfaces, 1989, 38, 149; Sagawa, K., Yolota, J., Ueno, I., Miyosi, T., Takehara, M. 1986 XIV Congress IFSCC*;* Tabohashi, T., Tobita, K., Sakomoto, K., Kouchi, J., Yokoyama, S., Sakai, H., Abe, M. Colloids and Surfactants B: Biointerfaces, 2001, 20, 79; Auberger, S., Garardin, C., Rodehüser, L., Finance, C., Nicolazzi, C, Pérez, L., Infante, M.R., Manresa, M.A., Selv, C., Progr. Colloid Polym Sci, 118, 145-158, 2001; Nnanna, I.A., Xia, J. Protein Based Surfactants: Synthesis, Physiochemical Properties and Applications, 2001 Marcel Dekker. NY). Since 1984, the surface-active agents synthesis group has been developing researches within this area, investigating the synthesis, study and development of monocatenary lipo-amino acids (amides, esters and acyls) of very different structures and ionic characteristics. These surface-active agents are characterised by having an amino acid or peptide in their hydrophilic or polar part and a fatty chain condensed to the amino acid via its amino or terminal carboxyl in its hydrophobic or apolar part. Similarly, by means of chemical and enzymatic methodologies, dicatenary lipo-amino acids and geminal compounds of very varied structure have been synthesised. These studies have given rise to a large number of patents and publications (ES 9500061 (1995); PI 9500027 (1995); PCT/ES96/00026 (1996); ES 9700520 (1997); ES 9900739 (1999); M.R. Infante, J. Molinero, P. Erra (1992), JAOCS, vol 69, No. 7; J. Molinero, M.R. Juliá, P. Erra, M. Robert; M.R. Infante, 1988, JAOCS, vol 65, No. 6; C. Solans, M.A. Pés, N. Azemar, M.R. Infante, 1990, Prog. Colloid Polym. Sci., 81 pp 144-150; L. Pérez, J.L. Torres, A. Manresa, C. Solans, M.R. Infante, 1996, Langmuir, 12, 229, pp 5296-5301; Clapés, P., Morán, C., Infante, M.R. 1999, Biotechnol. Bioeng. 63, 333).

Moreover, glycerolipids (mono- and diacylglycerides) currently constitute (Zhu, Y., Masuyama, A., Kirito, Y., Okahara, M., Rosen M.J. J. Am. Oil. Chem. Soc. 1992, 69, 6269) 75% of emulsifiers used in the food industry due to the fact that they are innocuous on account of being natural products and the fact that they possess excellent interfacial and vehicularising properties. The literature describes their properties in great detail (K. Larsson, 1994, "Lipids: Molecular Organization, Physical Functions and Technical Applications" The Oily Press LTD). It is usual in the industry to use these compounds in combination with natural lecithins and also with other emulsifiers of a more polar nature. On the other hand, the constant change in world tendencies in food (products low in calories, rich in vitamins or other elements) make it necessary to develop new emulsifiers which will permit these products to be manufactured. For these reasons, wide-scale use is being made of modified monoglycerides in which a chemical function has been introduced that causes their physico-chemical and solubility characteristics to alter in order to make them suitable for the new food formulations (Lipid Technologies), for example when the free hydroxyl end/s of the molecule are functionalised with organic acids of the lactic, citric and acetic acid type ("Food Emulsions" 1997, Ed, by Stig E. Friburg and K. Larsson)

The present invention seeks to carry out the synthesis of a novel family of acylglycerides (mono and di acyl) derived from amino acid lysine. These compounds consist of the amino acid lysine attached to one of two units of glycidol by means of an amine bond via the side amino group of the amino acid. The fatty chains are attached to the hydroxyl groups of the dihydrocypropyl chain by means of an ester bond.

Also recently synthesised are surface-active agents with a glycerolipid structure derived from the condensation of pure mono- and diglycerides with amino acids of the type arginine, acetyl-arginine, glutamic acid, aspartic acid, glutamine, asparagine and tyrosine. Morán, C., Infante, M.R., Clapés, P. J, Soc. Perkin Trans 1, 2001, 2063; Morán, C., Infante, M.R., Clapés, P. J, Soc. Perkin Trans 1, 2002, 1124; Pérez et al. New J. Chem. 26, 2002, 1221; Sunil A. David et al Bioorg Med Chem Lett, 2002, 12, 357; Pérez, P., Infante, M.R., Pons, R., Vinardell, M.P., Mitjans, M., Pinazo, A. Colloids and Surfaces B, 35, 2004, 235; Moran, C., Pinazo, A., Pérez, L., Clapés, P., Pons, R., Infante, M.R., Chimie, 7, 169, 2004; Pérez, L., Infante, M.R., Angelet, M., Clapés, P., Pinazo, A., Progr. Colloid Poly. Sci, 123, 2003; Infante, M.R., Pérez, L., Pinazo, A., Clapés, P., Morán, C., Angelet, M., Garcia, M.T., Vinardell, M.P. Chimie, 7, 2004, 583.

Given the structural similarity between the molecules forming the object of this patent and analogues derived from amino acids of the type arginine, acetyl-arginine, glutamic acid, aspartic acid, glutamine, asparagine and tyrosine which display surface-active and antimicrobial properties, the new derivatives of lysine will be able to be applied as cationic antimicrobial surface-active agents with emulsifying, dispersant and solubilising properties and/or controlled release agents.

A thorough examination of the chemical stability of the derivatives of arginine and acetyl-arginine reveals that this type of molecule displays hydrolysis of the amide bond between the glycerol skeleton and the amino acid arginine in relatively short times in aqueous solutions (Pérez, P., Infante, M.R. Pons, R., Vinardell, M.P., Mitjans, M., Pinazo, A. Colloids and Surfaces B, 35, 2004, 235).

The synthesis of compounds of the type N -n-acyloxypropyl lysine methyl ester and N,N -bis (n-acyloxypropyl) lysine methyl ester, the object of the present invention, requires the prior obtaining of the derivative N (2,3- dihydroxypropyl)L-lysine. The literature describes the synthesis of N (2,3- dihydroxypropyl)L-lysine (Lilota, A., K. Gerhard, Biological Chemistry Hoppe-Seyler, 1987, 11, 368) starting from glyceraldehyde and the amino acid lysine.

In relation to the acylation of the hydroxyl groups of the molecule, the use has been described of acid and basic catalysts (Rejzek, M., Vacek, M., Wimmer, Z., Helvetica Chimica Acta, 2000, 83, 2756; Gaffney, P.R. and Reese, C.B. J. Chem. Soc. Perkin Trans. I, 2001, 92) and also of lipase type enzymes (Valivety, R., Gill, I.S., Vulfson, E.N. 1998, J. Surf. Deterg. 1. 177-185).

The main novelties provided by the present invention are the combination in the same molecule of surface-active agents derived from the amino acid lysine and mono- and diglyceride surface-active agents which give rise to ionic and/or non-ionic mono- and diacyl glycerides, soluble in water and having wide industrial application. The introduction of the amino acid lysine by means of an amine bond will grant the molecule high chemical stability, a property necessary for certain industrial applications in which high temperatures and alkaline pH are required.

### DESCRIPTION OF THE INVENTION

The present invention refers to novel compounds having an amphiphilic character (cationic, anionic, amphoteric and non-ionic) derivatives of n acyloxypropyl-type lysine amino acid according to general formula (I), designed to be used in the food, pharmaceutical and cosmetic industries as surface-active agents having a self-aggregating capacity and antimicrobial properties. 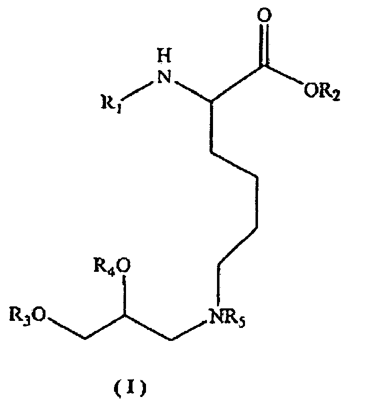 Where:
- R₁ can be a hydrogen or an acetyl (Ac) group or a protector group.
- R₂ can be a hydrogen, a methyl group, a saturated hydrocarbon chain or a cationic contraion.
- R₃, R₄ can be a hydrogen or a linear chain acyl (Ac) group, preferably saturated or unsaturated.
- R₅ can be a hydrogen or a CH₂CHOR₆CH₂OR₇ group, where R₆ and R₇ can be a hydrogen or a linear chain acyl group, preferably saturated or not.
- R₁, R₂, R₃, R₄, R₅, R₆ and R₇ cannot all be hydrogens at the same time.
The starting materials can be:
Derivatives of lysine, of technical quality or specifically N^{α}-acetyl (Ac), N^{α}-Benzyloxycarbonyl (Z) and
N^{α}-tert-butyloxycarbonyl (Boc) lysine. Glycidol, enantiomerically pure or the racemic mixture.
Acids, esters or chlorides of saturated or unsaturated linear fatty acids of different length.

In the molecules, the number of alkyl chains, the degree of unsaturation of the alkyl chains and the length of them will all be varied, which will give rise to compounds with different behaviour in the physico-chemical properties of adsorption, self-aggregation and in their biological properties.

The obtaining of these compounds has been carried out by chemical methodology. Described below are the stages involved in each of the procedures:
The synthesis of these compounds took place in four stages:
   1) Formation of the N- -protected esters of lysine 1-O-N-(prot)-lysine (which will be designated as (prot)K). As raw materials, thionyl chloride, methanol and L-lysine pure or its racemic mixtures were used with the N group protected.
   2) Formation of the derivatives N, O -protected N-dihydroxypropyl lysine (designated as (00)ₓ(prot)K where x can be 1 or 2). The reaction takes place starting from (prot)K which reacts with the glycidol in methanol solution.
   3) Formation of the acylated derivatives (nn)ₓ(prot)K. The reaction takes place starting from (00)ₓ(prot)K using chlorides of linear fatty acids with 8 to 14 carbon atoms as acylating agents in a basic organic medium.
   4) Formation of the compounds N -n-acyloxypropyl lysine methyl ester (which will be designated as (nn)ₓK) by means of a Pd/C catalytic hydrogenation or an acidolysis.

The present invention refers to surface-active agents of the type N-n-acyloxypropyl lysine methyl ester derived from the amino acid lysine of the type designed for acting as surface agents with antimicrobial activity. The variations in activity will be a function of the number of fatty chains and their length.

The purification of intermediate and final products is carried out by means of liquid/liquid and liquid/solid extractions, crystallisations, cationic exchange chromatography and normal phase chromatography.

### EXAMPLE OF EMBODIMENT OF THE INVENTION

By way of example and without limiting the scope of the procedure, given below are details of an example of obtaining the compound with fatty chains of 12 carbon atoms: N, N-bis (2-hydroxy, 3-lauroyloxy propyl) lysine methyl ester.

The compound is prepared in four stages as mentioned above.
1) *Preparation of Z-K*. A 0.3 molar solution is prepared of Z-Lysine-OH in methanol is prepared. It is chilled to -80°C and 5.8% by volume of thionyl chloride is added. The reaction is left for 20 hours. The product is isolated by means of successive evaporations of the solvent and of the excess thionyl chloride.
*2) Preparation of the product* (00)₂Z-K. A 0.04 molar solution of Z-K is prepared in dry methanol. 0.6% by volume of triethylamine is added. To this mixture glycidol is slowly added at a concentration of 0.09 M. The reaction mixture is kept stirring for 40 hours at 70°C. The solvent is then eliminated under vacuum. The product is obtained by means of purification by cationic exchange chromatography.
*3) Preparation of the product* (12 0)₂Z-K. A 0.1 molar solution of (00)₂Z-K is prepared in pyridine. 5.4% by volume of lauroyl chloride is added. The mixture is left to react for 8 hours at room temperature. After eliminating the pyridine under vacuum, the product is purified in column chromatography by means of silica using mixtures of increasing polarity of chloroform:methanol as eluents.
3) *Preparation of the (12 0)₂K*. A 0.1 molar solution of (12 0)₂Z-K is prepared in MeOH. 10% by weight of the catalyst Pd/C is added. A pressure of 600 psi of nitrogen is applied for 1 hour at room temperature. The product is obtained by elimination of the catalyst filtering the mixture through celite and evaporating the solvent.

## Claims

1. Compounds of the type N-n-acyloxypropyl lysine methyl ester and N,N-bis(n-acyloxypropyl) lysine methyl ester **characterised by** the general formula: 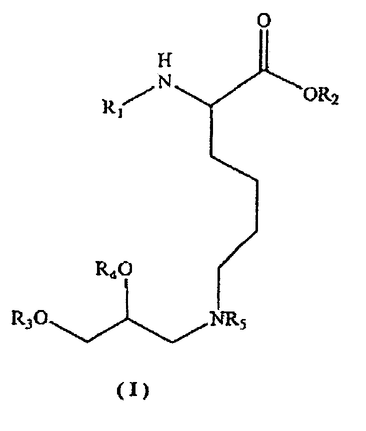 Where:
- R₁ can be a hydrogen or an acetyl (Ac) group or a protector group.
- R₂ can be a hydrogen, a methyl group, a saturated hydrocarbon chain or a cationic contraion.
- R₃, R₄ can be a hydrogen or a linear chain acyl (Ac) group, preferably saturated or unsaturated.
- R₅ can a hydrogen or a group CH₂CHOR₆CH₂OR₇, where R₆ and R₇ can be a hydrogen or a linear chain acyl group, preferably saturated or not.
- R₁, R₂, R₃, R₄, R₅, R₆ and R₇ cannot all be hydrogens at the same time.

2. Procedure for obtaining amphiphilic compounds of general formula according to claim 1, **characterised by** the following stages:
- Formation of esters of lysine 1-O-N-(prot)-lysine (which will be designated as (prot)K) using as raw materials thionyl chloride and L-lysine protected by the N group.
- Formation of the derivatives N, O -protected N - dihydroxypropyl lysine (designated as (00)ₓ(prot)K).
- Formation of the protected acylated derivatives N -n-acyloxypropyl lysine (which shall be designated (nn)ₓK) starting from (00)ₓ(prot) using chlorides of linear fatty acids with from x to w carbon atoms as acylating agents in a pyridine medium.
- Formation of the acylated derivatives N -n-acyloxypropyl lysine (which shall be designated (nn)ₓK) by means of a Pd/C catalytic hydrogenation.

3. A procedure according to claim 1 and 2, **characterised by** using L-lysine, pure or its racemic mixtures, as starting compounds.

4. A procedure according to claim 1 and 2, **characterised by** using as protector groups of the -amino function of the lysine, the groups acetyl (Ac), benzyloxycarbonyl (Z) and tert-butyloxycarbonyl (Boc).

5. A procedure according to claim 1 and 2, in which the first stage of the procedure is **characterised by** obtaining compounds N -dihydroxypropyl lysine starting from N-protected lysine and glycidol.

6. A procedure for obtention according to claim 1, 2 and 5, **characterised by** using glycidol, enantiomerically pure or the racemic mixture.

7. A procedure according to claim 1 and 2, in which the second stage of the procedure is **characterised by** the acylation of the free hydroxyl groups of N -dihydroxypropyl lysine using pyridine as reaction medium.

8. A procedure according to claim 1, 2 and 7, **characterised by** the use of chlorides of fatty acids with linear chains of 8, 10, 12, 14 carbon atoms, saturated or unsaturated, in the acylation reaction of the free hydroxyl groups of N-dihydroxypropyl lysine.

9. A procedure according to claim 1 and 2, in which optionally and if necessary the deprotection of the - amino group of the lysine is carried out by a catalytic hydrogenation with Pd/C.

10. A procedure according to claim 1, and 2, **characterised in that** the monitoring of the reaction is done by means of high performance liquid chromatography, with a propylcyan type column, using water and acetonitrile as eluents.

11. Use of the compound of the type N-n-acyloxypropyl lysine methyl ester and N,N-bis(n-acyloxypropyl) lysine methyl ester, according to claim 1, **characterised by** its antimicrobial properties.

12. Use of the compound of the type N -n-acyloxypropyl lysine methyl ester and N,N-bis(n-acyloxypropyl) lysine methyl ester, according to claim 1, **characterised by** its n-acylglyceride aggregation properties.

13. Use of the compound of the type N -n-acyloxypropyl lysine methyl ester and N,N-bis(n-acyloxypropyl) lysine methyl ester, according to claim 1, as cationic, anionic, zwitterion and/or non-ionic surface-active agent.

14. Use of the compound of the type N -n-acyloxypropyl lysine methyl ester and N,N-bis(n-acyloxypropyl) lysine methyl ester, according to claim 1, as high surface-active agents with emulsifying and vehicularising properties and/or with antimicrobial activity.
